(19) **Europäisches Patentamt / European Patent Office / Office européen des brevets**

(11) **EP 2 062 460 B1**

(12) **EUROPEAN PATENT SPECIFICATION**

(45) Date of publication and mention
of the grant of the patent:
**16.03.2016 Bulletin 2016/11**

(21) Application number: **06815899.7**

(22) Date of filing: **29.09.2006**

(51) Int Cl.:
**H05B 3/34** *(2006.01)*      *A61F 7/00* *(2006.01)*

(86) International application number:
**PCT/US2006/038231**

(87) International publication number:
**WO 2008/033147 (20.03.2008 Gazette 2008/12)**

(54) **HEATING BLANKET**

HEIZDECKE

COUVERTURE CHAUFFANTE

(84) Designated Contracting States:
**AT BE BG CH CY CZ DE DK EE ES FI FR GB GR HU IE IS IT LI LT LU LV MC NL PL PT RO SE SI SK TR**

(30) Priority: **13.09.2006 US 825573 P**

(43) Date of publication of application:
**27.05.2009 Bulletin 2009/22**

(73) Proprietor: **Augustine Biomedical and Design, LLC**
**Eden Prairie, MI 55344 (US)**

(72) Inventors:
• **AUGUSTINE, Scott, D.**
**Bloomington, MN 55438 (US)**
• **ARNOLD, Randall, C.**
**Minnetonka, MN 55305 (US)**
• **AUGUSTINE, Ryan, S.**
**Minneapolis, MN 55410 (US)**
• **DEIBEL, Rudolf, A.**
**Eden Prairie, MN 55346 (US)**

• **ENTENMAN, Scott, A.**
**St. Paul, MN 55102 (US)**
• **LAWRENCE, Gordon, D.**
**Minneapolis, MN 55417 (US)**
• **LELAND, Keith, J.**
**Medina, MN 55340 (US)**
• **NEILS, Thomas, F.**
**Minneapolis, MN 55408 (US)**

(74) Representative: **Howe, Steven**
**Reddie & Grose LLP**
**16 Theobalds Road**
**London WC1X 8PL (GB)**

(56) References cited:
WO-A1-99/25155     GB-A- 969 253
US-A- 3 808 403     US-A- 4 149 066
US-A- 4 186 294     US-A- 5 023 433
US-A1- 2002 117 494     US-B1- 6 172 344
US-B1- 7 053 344

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

TECHNICAL FIELD

The present invention is related to heating or warming blankets or pads and more particularly to those including electrical heating elements.

BACKGROUND

It is well established that surgical patients under anesthesia become poikilothermic. This means that the patients lose their ability to control their body temperature and will take on or lose heat depending on the temperature of the environment. Since modem operating rooms are all air conditioned to a relatively low temperature for surgeon comfort, the majority of patients undergoing general anesthesia will lose heat and become clinically hypothermic if not warmed.

Over the past 15 years, forced-air warming (FAW) has become the "standard of care" for preventing and treating the hypothermia caused by anesthesia and surgery. FAW consists of a large heater/blower attached by a hose to an inflatable air blanket. The warm air is distributed over the patient within the chambers of the blanket and then is exhausted onto the patient through holes in the bottom surface of the blanket.

Although FAW is clinically effective, it suffers from several problems including: a relatively high price; air blowing in the operating room, which can be noisy and can potentially contaminate the surgical field; and bulkiness, which, at times, may obscure the view of the surgeon. Moreover, the low specific heat of air and the rapid loss of heat from air require that the temperature of the air, as it leaves the hose, be dangerously high - in some products as high as 45°C. This poses significant dangers for the patient. Second and third degree bums have occurred both because of contact between the hose and the patient's skin, and by blowing hot air directly from the hose onto the skin without connecting a blanket to the hose. This condition is common enough to have its own name - "hosing." The manufacturers of forced air warming equipment actively warn their users against hosing and the risks it poses to the patient.

To overcome the aforementioned problems with FAW, several companies have developed electric warming blankets. However, these electric blankets have a number of inadequacies, for example, the risk of heat and pressure injuries that may be suffered by a patient improperly coming into contact with the electrical heating elements of these blankets. It is well established that heat and pressure applied to the skin can rapidly cause thermal injury to that skin. Such contact may arise if a patient inadvertently lies on an edge of a heated blanket, if a clinician improperly positions an anesthetized patient atop a portion of the heated blanket, or if a clinician tucks an edge of the blanket about the patient. Thus, there is a need for a heating blanket that effectively forms a cocoon about a patient, in order to provide maximum efficacy in heating, without posing the risk of burning the patient.

There is also a need for electrically heated blankets or pads that can be used safely and effectively warm patients undergoing surgery or other medical treatments. These blankets need to be flexible in order to effectively drape over the patient (making excellent contact for conductive heat transfer and maximizing the area of the patient's skin receiving conductive as well as radiant heat transfer), and should incorporate means for precise temperature control.

Precise temperature control is important because non-uniform heat distribution can occur within an electric warming blanket. Unfortunately, many temperature sensors used to provide feedback to a temperature controller do not dependably report an accurate average temperature of the blanket because they sense temperature from too small of an area. For example, if the temperature of a measured location is cooler than the average blanket temperature, the temperature sensor will cause the controller to deliver more power to the heater and the resulting average temperature of the heater will be higher than desired.

Further, an electric blanket can overheat if the temperature sensor is thermally grounded to a cool object. This condition can occur if a cool object such as a metal pan is placed on top of the heater in the area of the temperature sensor. The sensor "feels" cool and tells the temperature controller to deliver more power to the heater.

Accordingly, there is a need for a blanket that utilizes a temperature sensor that takes temperature measurements that are representative of the average temperature of the blanket. Further, there is a need for a blanket with a temperature sensor that will not cause the blanket to overheat if a cool object is placed in proximity to it. Various embodiments of the invention described herein solve one or more of the problems discussed above.

Electric warming blankets overcome the aforementioned problems with FAW. Some of these warming blankets employ flexible heaters, which may be prone to potentially dangerous conditions, for example, when the blankets, including the flexible heaters, are folded over onto themselves. Such folding, which is sometimes called "rucking", may result in electrical shorting between portions of the flexible heater. The short circuit becomes a relatively low-resistance pathway and current will preferentially flow through the low resistance area. The increased current flow may cause that area to get very hot which may cause a burn risk to the patient.

Electrical shorting with such heaters has been addressed by electrically insulating the heater by laminating a relatively thick layer of plastic film to each side of the heater. However, when electrical insulation is accomplished by laminating a relatively thick layer of plastic film to each side of the fabric heater, the resulting laminated structure becomes relatively stiff and non-flexible and does not exhibit desirable draping characteristics. A non-flexible, non-draping blanket is not only uncomfortable for the patient, but is also thermally inefficient because of the poor thermal contact with the patient. Non-flexible thermal blankets can also apply excessive heat and pressure to patient "high spots," such as boney prominences.

Further, rucking may cause overheating of the flexible heater due to added thermal insulation over the side of the heater that is beneath a folded-over portion of the heater. Normally, the heater will lose heat off of both surfaces simultaneously. If the heater is folded back on itself, the upper layer of heater becomes a very effective guard heater. This near perfect thermal insulation on the upper side prevents the lower layer (e.g., the patient side) from losing heat to the environment. Therefore, the temperature of the lower of the two layers will increase to a new and higher equilibrium temperature. If the heater is folded like a "Z" so that there is an area that is three layers thick, the middle layer of the "Z" will not be able to lose heat from either of its surfaces. The area in the middle of the three-layer fold will significantly over-heat and may become unsafe.

A traditional approach to avoiding electrical shorting and/or overheating has been to purposefully make the blanket relatively stiff in order to prevent rucking. This stiffening is typically accomplished by laminating the heater material to plastic film or enclosing the heater in a relatively stiff outer cover. As previously discussed, stiff blankets may be uncomfortable for a patient and may be less efficient in heating the patient, since the stiffness prevents a draping of the blankets over the patient to maximize an area of the patient's skin receiving conductive as well as radiant heat transfer. Accordingly, there is a need for a blanket that an avoid electrical shorting and/or overheating caused by rucking without becoming so stiff as to lose desirable draping characteristics. Various embodiments of the invention described herein solve one or more of the problems discussed above.

Electric warming blankets overcome the aforementioned problems with FAW. Some of these warming blankets employ flexible conductive fabric heaters, the flexibility of which is desirable to maintain when employing in the blankets. In applications such as these, where the heater is subject to flexing, couplings directly between the fabric heater and bus bars, which extend along opposing edges of the heater to supply power to the heater, may be susceptible to zones of intermittent contact along a length of each of the bus bars. Thus there is a need for flexible heater subassemblies that include bus bar couplings which are not susceptible to zones of intermittent contact. There is also a need for flexible heater subassemblies wherein interfaces between bus bars and heaters are augmented to facilitate more uniform contact therebetween.

The standard method of coupling the electrical power supply to any large heater surface is to place a metal bus bar conductor near two of the opposing edges of the heater. Electrical power flows from the power supply through the bus bars and is evenly distributed along the entire length of the heater. The electrically conductive bus bar material contacts the electrically conductive heater and the current flows between the two materials. Unfortunately, the conductive bus bars do not make a dependable, uniform and stable connection to the conductive heater, especially during flexing of the heater, because both the heater and the bus bar are flexible. Generally, two flexible pieces of material that are placed together will not maintain reliable and uniform contact across their entire surface, especially during repeated flexing.

When the bus bar/heater interface is flexed, the heater temporarily separates slightly from the bus bar at point locations. This separation prevents current from flowing at the separation point, forcing the current that would have passed through that point to flow instead through the adjacent points that are still in contact. The increased current flowing through the adjacent points can cause those points to over-heat. Repeated over-heating can cause the heater at that point to eventually fail and stop conducting electricity. When a point fails, it is permanently removed from the current path and the adjacent points must pick up the extra flow. The extra flow caused by the failed point, in addition to the extra flow caused by the areas of non-contact due to flexion, may result in over-heating and failure of the remaining points.

Accordingly, there remains a need for flexible heater subassemblies and blankets that allow the bus bar and the heater to be coupled in such a manner that current can be dependably and uniformly supplied from the bus bar to the heater without potentially patient harming blanket over-heating and/or failure. Various embodiments of the invention described herein solve one or more of the problems discussed above.

US 4,149,066 describes a flexible heat-emitting blanket. The blanket includes a sheet-like thin flexible heat-emitting surface and a flexible serpentine heat-sensing layer.

US 4,186,294 describes a therapeutic heating pad which uses infrared radiation to warm a user. The pad includes a radiant heat generating layer covered by flexible radiation permeable layers.

US 3,808,403 describes flexible heating sheets. The sheets are prepared by applying a thin layer of conductive paint to a flexible sheet.

GB 969,253 describes flexible waterproof enclosures for heating pads.

US 2002/0117494 describes electric heating elements which include an electrical heating circuit attached to the inner surface of a water-resistant, vapour permeable bladder.

WO 99/25155 describes a heat producing cover for spectators at outdoor events. The cover includes an electrically powered heating element and a controller.

SUMMARY OF THE INVENTION

The invention is defined in claim 1, to which reference is now directed.

BRIEF DESCRIPTION OF THE DRAWINGS

The following drawings are illustrative of particular embodiments of the present invention and therefore do not limit the scope of the invention. The drawings are not to scale (unless so stated) and are intended for use in conjunction with the explanations in the following detailed description. Embodiments of the present invention will hereinafter be described in conjunction with the appended drawings, wherein like numerals denote like elements.

Figure 1A is a plan view of a flexible heating blanket subassembly for a heating blanket, according to some embodiments of the present invention.
Figures 1B-C are end views of two embodiments of the subassembly shown in Figure 1A.
Figure 1D is a schematic showing a blanket including the subassembly of Figure 1A draped over a body.
Figure 2A is a top plan view of a heating element assembly, according to some embodiments of the present invention, which may be incorporated in the blanket shown in Figure 3A.
Figure 2B is a section view through section line A-A of Figure 2A.
Figure 2C is an enlarged plan view and corresponding end view schematic of a portion of the assembly shown in Figure 2A, according to some embodiments of the present invention.
Figure 2D is an enlarged view of a portion of the assembly shown in Figure 2A, according to some embodiments of the present invention.
Figure 3A is a top plan view, including partial cutaway views, of a lower body heating blanket, according to some embodiments of the present invention.
Figure 3B is a schematic side view of the blanket of Figure 3A draped over a lower body portion of a patient.
Figure 3C is a top plan view of a heating element assembly, which may be incorporated in the blanket shown in Figure 3A
Figure 3D is a cross-section view through section line D-D of Figure 3C.
Figure 4A is a plan view of flexible heating element, according to some alternate embodiments of the present invention.
Figure 4B is a top plan view, including a partial cutaway view, of a heating element assembly, according to some embodiments of the present invention, which may be incorporated in the blanket shown in Figure 4C.
Figure 4C is a top plan view, including a partial cutaway view, of an upper body heating blanket, according to some embodiments of the present invention.
Figure 4D is a schematic end view of the blanket of Figure 4B draped over an upper body portion of a patient.

DETAILED DESCRIPTION

The following detailed description is exemplary in nature and is not intended to limit the scope, applicability, or configuration of the invention in any way. Rather, the following description provides practical illustrations for implementing exemplary embodiments of the present invention. Examples of constructions, materials, dimensions, and manufacturing processes are provided for selected elements, and all other elements employ that which is known to those of skill in the field of the invention. Those skilled in the art will recognize that many of the examples provided have suitable alternatives that can be utilized. The term 'blanket', used to describe embodiments of the present invention, may be considered to encompass heating blankets and pads.

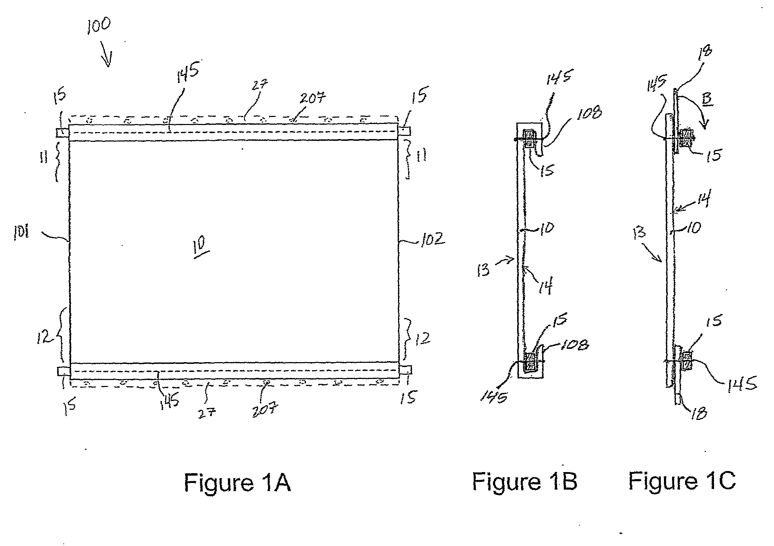

Figure 1A is a plan view of a flexible heating blanket subassembly 100, according to some embodiments of the present invention; and Figures 1B-C are end views of two embodiments of the subassembly shown in Figure 1A. Figure 1A illustrates a flexible sheet-like heating element, or heater 10 of subassembly 100 including a first end 101, a second end 102, a first lateral portion 11 extending between ends 101, 102, and a second lateral portion 12, opposite first lateral portion 11, also extending between ends 101, 102. Heater 10 comprises a conductive fabric or a fabric incorporating closely spaced conductive elements such that heater 10 has a substantially uniform watt density output, preferably less than approximately 0.5 watts/sq. inch, and more preferably between approximately 0.2 and approximately 0.4 watts/sq. inch, across a surface area, of one or both sides 13, 14 (Figures 1B-C), the surface area including and extending between lateral portions 11, 12 of heater 10. Some examples of conductive fabrics which may be employed by embodiments of the present invention include, without limitation, carbon fiber fabrics, fabrics made from carbonized fibers, woven or non-woven non-conductive substrates coated with a conductive material, for example, polypyrrole, carbonized ink, or metalized ink.

Figure 1A further illustrates subassembly 100 including two bus bars 15 coupled to heating element 10 for powering element 10; each bar 15 is shown extending alongside opposing lateral portions 11, 12, between first and second ends 101, 102. With reference to Figure 1B, according to some embodiments, bus bars 15 are coupled to heating element 10 within folds of opposing wrapped perimeter edges 108 of heating element 10 by a stitched coupling 145, for example, formed with conductive thread such as silver-coated polyester or nylon thread (Marktek Inc., Chesterfield, MO), extending through edges 108 of heating element 10, bars 15, and again through heating element 10 on opposite side of bars 15. According to alternate embodiments heating element 10 is not folded over bus bars 15 as shown. Alternative threads or yarns employed by embodiments of the present invention may be made of other polymeric or natural fibers coated with other electrically conductive materials; in addition, nickel, gold, platinum and various conductive polymers can be used to make conductive threads. Metal threads such as stainless steel, copper or nickel could also be used for this application. According to an exemplary embodiment, bars 15 are comprised of flattened tubes of braided wires, such as are known to those skilled in the art, for example, a flat braided silver coated copper wire, and may thus accommodate the thread extending therethrough, passing through openings between the braided wires thereof. In addition such bars are flexible to enhance the flexibility of blanket subassembly 100. According to alternate embodiments, bus bars 15 can be a conductive foil or wire, flattened braided wires not formed in tubes, an embroidery of conductive thread, or a printing of conductive ink. Preferably, bus bars 15 are each a flat braided silver-coated copper wire material, since a silver coating has shown superior durability with repeated flexion, as compared to tin-coated wire, for example, and may be less susceptible to oxidative interaction with a polypyrrole coating of heating element 10 according to an embodiment described below. Additionally, an oxidative potential, related to dissimilar metals in contact with one another is reduced if a silver-coated thread is used for stitched coupling 145 of a silver-coated bus bar 15.

According to some preferred embodiments, two or more rows of stitches are applied to each bus bar 15 for added safety and stability of the bus bar/heating element interface. Preferably, the two rows of stitches are oriented in a "zigzag" pattern so that each row of stitches captures an edge of bus bar 15. A zigzag pattern of relatively closely positioned stitches stabilizes flexible heating element 10 and holds it in close opposition to bus bar 15 so that the fabric cannot physically pull away from the bus bar during flexing. According to some additional embodiments, a ribbon of highly conductive material is interposed between bus bar 15 and heating element 10. For example, a ribbon of cloth that has been coated with a conductive metal such as silver works very well in this application. The cloth ribbon is soft, flexible and fibrous and therefore integrates itself into the fibrous matrix of both bus bar 15 and heating element 10. Other options for improving the electrical connection between bus bar 15 and heating element 10 include a ribbon of highly conductive paint or ink, applied to the conductive fabric of heating element 10, to which bus bar 15 is attached rather than directly to the conductive fabric of heating element 10.

According to an exemplary embodiment, a conductive fabric comprising heating element 10 comprises a non-woven polyester having a basis weight of approximately 130 g/m$^2$ and being 100% coated with polypyrrole (available from Eeonyx Inc., Pinole, CA); the coated fabric has an average resistance, for example, determined with a four point probe measurement, of approximately 15-20 ohms per square inch at about 48 volts, which is suitable to produce the preferred watt density of 0.2 to 0.4 watts/sq.in. for surface areas of heating element 10 having a width, between bus bars 15, in the neighborhood of about 20 inches. Such a width is suitable for a lower body heating blanket, some embodiments of which will be described below. A resistance of such a conductive fabric may be tailored for different widths between bus bars (wider requiring a lower resistance and narrower requiring a higher resistance) by increasing or decreasing a surface area of the fabric that can receive the conductive coating, for example by increasing or decreasing the basis weight of the fabric. Resistance over the surface area of the conductive fabrics is generally uniform in many embodiments of the present invention. However, the resistance over different portions of the surface area of conductive fabrics such as these may vary, for example, due to variation in a thickness of a conductive coating, variation within the conductive coating itself, variation in effective surface area of the substrate which is available to receive the conductive coating, or variation in the density of the substrate itself. Local surface resistance across a heating element, for example heater 10, is directly related to heat generation according to the following relationship:

$$Q \,(\text{Joules}) = I^2 (\text{Amps}) \times R (\text{Ohms})$$

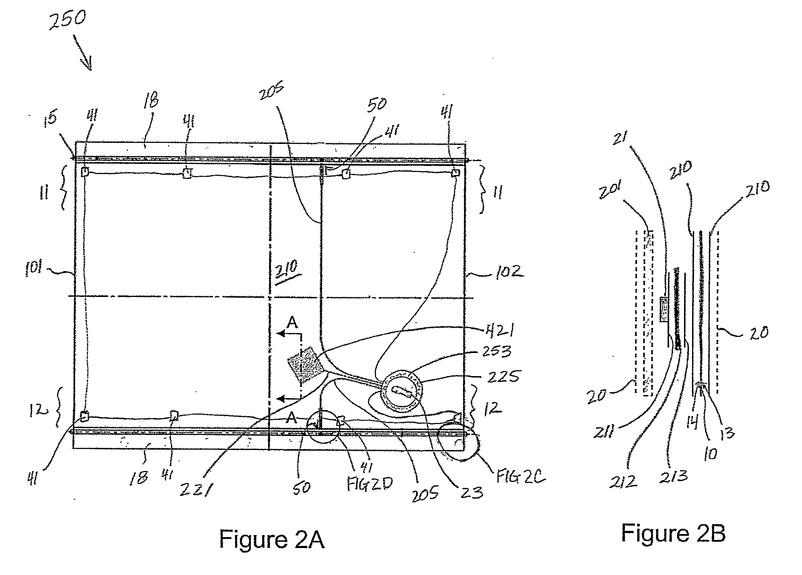

Variability in resistance thus translates into variability in heat generation, which is measured as a temperature. According to preferred embodiments of the present invention, which are employed to warm patients undergoing surgery, precise temperature control is desirable. Means for determining heating element temperatures, which average out temperature variability caused by resistance variability across a surface of the heating element, are described below in conjunction with Figures 2A-B.

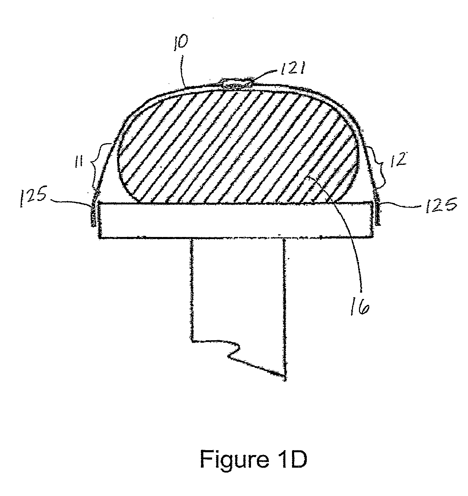

A flexibility of blanket subassembly 100, provided primarily by flexible heating element 10, and optionally enhanced by the incorporation of flexible bus bars, allows blanket subassembly 100 to conform to the contours of a body, for example, all or a portion of a patient undergoing surgery, rather than simply bridging across high spots of the body; such conformance may optimize a conductive heat transfer from element 10 to a surface of the body. However, as illustrated in Figure 1D, heating element 10 may be draped over a body 16 such that lateral portions 11, 12 do not contact side surfaces of body16; the mechanism of heat transfer between portions 11, 12 and body 16, as illustrated in Figure 1D, is primarily radiant with some convection.

The uniform watt-density output across the surface areas of preferred embodiments of heating element 10 translates into generally uniform heating of the surface areas, but not necessarily a uniform temperature. At locations of heating element 10 which are in conductive contact with a body acting as a heat sink, for example, body 16, the heat is efficiently drawn away from heating element 10 and into the body, for example by blood flow, while at those locations where element 10 does not come into conductive contact with the body, for example lateral portions 11, 12 as illustrated in Figure 1D, an insulating air gap exists between the body and those portions, so that the heat is not drawn off those portions as easily. Therefore, those portions of heating element 10 not in conductive contact with the body will gain in temperature, since heat is not transferred as efficiently from these portions as from those in conductive contact with the body. The 'non-contacting' portions will reach a higher equilibrium temperature than that of the 'contacting' portions, when the radiant and convective heat loss equal the constant heat production through heating element 10. Although radiant and convective heat transfer are more efficient at higher heater temperatures, the laws of thermodynamics dictate that as long as there is a uniform watt-density of heat production, even at the higher temperature, the radiant and convective heat transfer from a blanket of this construction will result in a lower heat flux to the skin than the heat flux caused by the conductive heat transfer at the 'contacting' portions at the lower temperature. Even though the temperature is higher, the watt-density is uniform and, since the radiant and convective heat transfer are less efficient than conductive heat transfer, the 'non-contacting' portions must have a lower heat flux. Therefore, by controlling the 'contacting' portions to a safe temperature, for example, via a temperature sensor 121 coupled to heating element 10 in a location where element 10 will be in conductive contact with the body, as illustrated in Figure 1D, the 'non-contacting' portions, for example, lateral portions 11, 12, will also be operating at a safe temperature because of the less efficient radiant and convective heat transfer. According to preferred embodiments, heating element 10 comprises a conductive fabric having a relatively small thermal mass so that when a portion of the heater that is operating at the higher temperature is touched, suddenly converting a 'non-contacting' portion into a 'contacting' portion, that portion will cool almost instantly to the lower operating temperature.

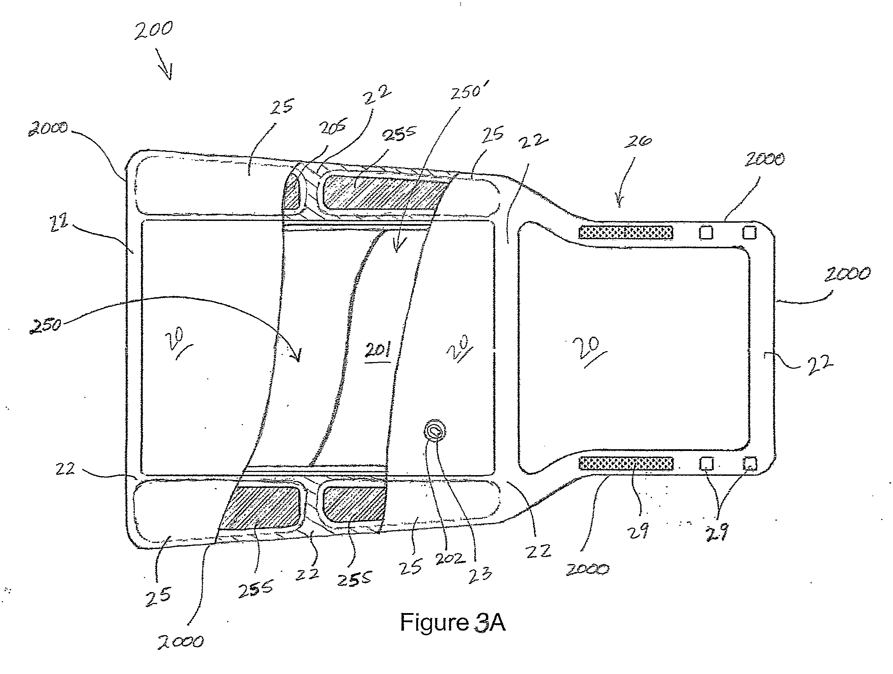

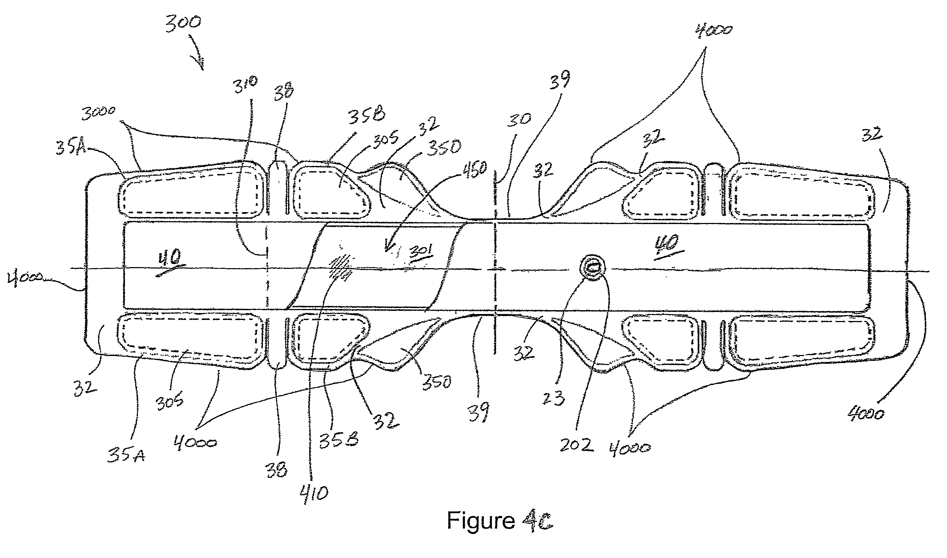

According to embodiments of the present invention, zones of heating element 10 may be differentiated according to whether or not portions of element 10 are in conductive contact with a body, for example, a patient undergoing surgery. In the case of conductive heating, gentle external pressure may be applied to a heating blanket including heating element 10, which pressure forces heating element 10 into better conductive contact with the patient to improve heat transfer. However, if excessive pressure is applied the blood flow to that skin may be reduced at the same time that the heat transfer is improved and this combination of heat and pressure to the skin can be dangerous. It is well known that patients with poor perfusion should not have prolonged contact with conductive heat in excess of approximately 42°C. 42°C has been shown in several studies to be the highest skin temperature, which cannot cause thermal damage to normally perfused skin, even with prolonged exposure. (Stoll & Greene, Relationship between pain and tissue damage due to thermal radiation. J. Applied Physiology 14(3):373-382. 1959. and Moritz and Henriques, Studies of thermal injury: The relative importance of time and surface temperature in the causation of cutaneous burns. Am. J. Pathology 23:695-720, 1947) Thus, according to certain embodiments of the present invention, the portion of heating element 10 that is in conductive contact with the patient is controlled to approximately 43°C in order to achieve a temperature of about 41-42°C on a surface a heating blanket cover that surrounds element 10, for example, a cover or shell 20, 40 which will be described below in conjunction with Figures 3A and 4C. With further reference to Figure 1D, flaps 125 are shown extending laterally from either side of heating element 10 in order to enclose the sides of body 16 thereby preventing heat loss; according to preferred embodiments of the present invention, flaps 125 are not heated and thus provide no thermal injury risk to body if they were to be tucked beneath sides of body 16.

Referring now to the end view of Figure 1C, an alternate embodiment to that shown in Figure 1B is presented. Figure 1C illustrates subassembly 100 wherein insulating members 18, for example, fiberglass material strips having an optional PTFE coating and a thickness of approximately 0.003 inch, extend between bus bars 15 and heating element 10 at each stitched coupling 145, so that electrical contact points between bars 15 and heating element 10 are solely defined by the conductive thread of stitched couplings 145. Alternatively, the electrical insulation material layer could be made of polymeric film, a polymeric film reinforced with a fibrous material, a cellulose material, a glass fibrous material, rubber sheeting, polymeric or rubber coated fabric or woven materials or any other suitable electrically insulating material. Each of the conductive thread stitches of coupling 145 maintains a stable and constant contact with bus bar 15 on one side and heating element 10 on the other side of insulator 18. Specifically, the stitches produce a stable contact in the face of any degree of flexion, so that the potential problem of intermittent contact between bus bar 15 and heating element 10 (that could arise for the embodiment shown in Figure 1B, where bus bar 15 is in physical contact with heating element 10) can be avoided. The stitches are the only electrical connection between bus bar 15 and heating element 10, but, since the conductive thread has a much lower electrical resistance than the conductive fabric of heating element 10, the thread does not heat under normal conditions. In addition to heating blanket applications described herein, such a design for providing for a uniform and stable conductive interface between a bus bar and a conductive fabric heater material can be used to improve the conductive interface between a bus bar or electrode and a conductive fabric in non-flexible heaters, in electronic shielding, in radar shielding and other applications of conductive fabrics.

Preferably, coupling 145 includes two or more rows of stitches for added security and stability. However, due to the flexible nature of blanket subassembly 100, the thread of stitched couplings 145, for either embodiment of Figure 1B or Figure 1C, may undergo stresses that, over time and with multiple uses of a blanket containing subassembly 100, could lead to one or more fractures along the length of stitching 145. Such a fracture, if it occurred in the embodiment of Figure 1B, could also result in intermittent contact points, between bus bar 15 and heating element 10, that could lead to a melt down of element 10 along bus bar. But, if such a fracture were to occur in the embodiment of Figure 1C, insulating member 18 may prevent a meltdown of element 10, so that only the conductive thread of stitching 145 melts down along bus bar 15.

Referring back to Figure 1A, bus bars 15 are shown extending past ends 101 and 102 of heating element 10, according to preferred embodiments. If bus bars did not extend at least to ends 101 and 102, increased current would flow from ends of bus bars 15 and into the fabric of heating element 10. Normally the current flows approximately perpendicularly between bus bars 15, therefore, each point on one of bus bars 15 supplies a narrow line of current to the other of bus bars 15. If either bus bar 15 terminates before reaching the end of the heater fabric, current will flow out the end of that bus bar. The excess current flow can result in excessive heating of the fabric of heating element 10, adjacent the end of that bus bar, which can cause degradation of the fabric leading to a catastrophic failure of heating element 10 by spreading along the entire bus bar. To avoid such a failure and to improve manufacturing reliability, both ends of bus bars 15 are extended beyond ends 101, 102 of heating element 10, preferably over a length of at least approximately ½ cm. According to these embodiments, the conductive thread stitches, previously described, also extend past ends 101, 102 being terminated on the bus bar extensions. This design advantageously creates an easy manufacturing process, which assures a dependable and repeatedly manufacturable bus bar termination which avoids the creation of hot spots at the ends of bus bars 15.

Figure 2A is a top plan view of a heating element assembly 250, according to some embodiments of the present invention, which may be incorporated by blanket 200, which is shown in Figure 3A and further described below. Figure 2B is a section view through section line A-A of Figure 2A. Figures 2A-B illustrate a temperature sensor assembly 421 assembled on side 14 of heating element and heating element 10 overlaid on both sides 13, 14 with an electrically insulating layer 210, preferably formed of a flexible non-woven high loft fibrous material, for example, 1.5 OSY (ounces per square yard) nylon, which is preferably laminated to sides 13, 14 with a hotmelt laminating adhesive. In some embodiments, the adhesive is applied over the entire interfaces between layer 210 and heating element 10. Other examples of suitable materials for layer 210 include, without limitation, polymeric foam, a woven fabric, such as cotton or fiberglass, and a relatively thin plastic film, cotton, and a non-flammable material, such as fiberglass or treated cotton. According to preferred embodiments, overlaid layers 210, without compromising the flexibility of heating assembly 250, prevent electrical shorting of one portion of heating element 10 with another portion of heating element 10 if heating element 10 is folded over onto itself. Heating element assembly 250 may be enclosed within a relatively durable and waterproof shell, for example shell 20 shown with dashed lines in Figure 2B, and will be powered by a relatively low voltage (approximately 48V). Layers 210 may even be porous in nature to further maintain the desired flexibility of assembly 250.

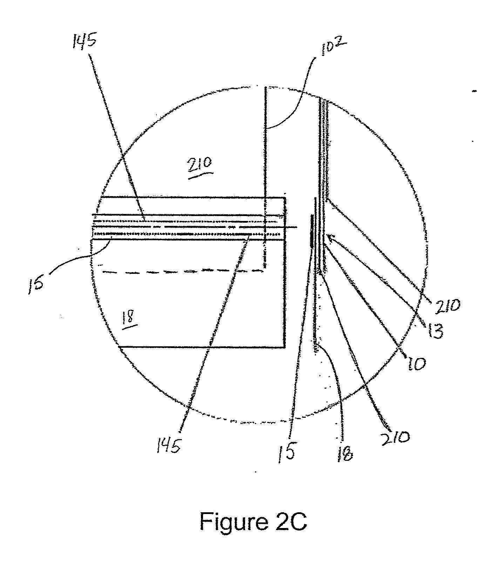

Figure 2C is an enlarged plan view and a corresponding end view schematic showing some details of the corner of assembly 250 that is circled in Figure 2A, according to some embodiments. Figure 2C is representative of each corner of assembly 250. Figure 2C illustrates insulating layer 210 disposed over side 14 of heating element and extending beneath bus bar 15, optional electrical insulating member 18, and layer 210 disposed over side 13 of heating element 10 and terminated adjacent bus bar 15 within lateral portion 12 so that threads of conductive stitching 145 securing bus bars 15 to heating element 10 electrically contact heating element 10 along side 13 of heating element 10. Figure 2C further illustrates two rows of conductive stitching 145 coupling bus bar 15 to heating element 10, and bus bar 15 and insulating member 18 extending past end 102; a backtack securing stitching 145 may be approximately 0.375 inches long and also extends beyond end 102.

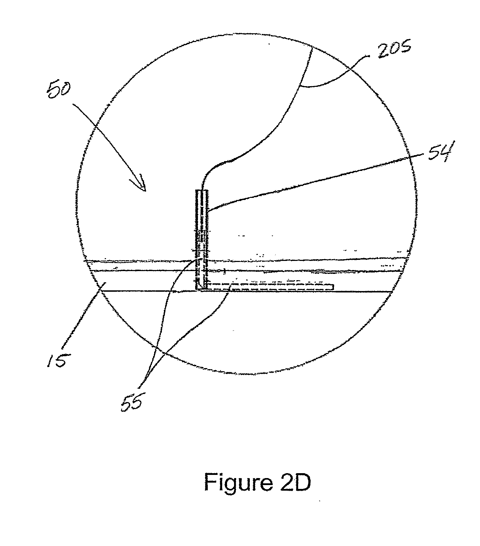

Figure 2A further illustrates junctions 50 coupling leads 205 to each bus bar 15, and another lead 221 coupled to and extending from temperature sensor assembly 421; each of leads 205, 221 extend over insulating layer 210 and into an electrical connector housing 225 containing a connector 23, which will be described in greater detail below, in conjunction with Figures 3A-C. Figure 2D is an enlarged view of junction 50, which is circled in Figure 2A, according to some embodiments of the present invention. Figure 2D illustrates junction 50 including a conductive insert 55 which has been secured to bus bar 15, for example, by inserting insert 55 through a side wall of bus bar 15 and into an inner diameter thereof, the bus bar 15 of the illustrated embodiment being formed by a braided wire tube so that an opening between the wires may be formed for access to the inner diameter. Insert 55 may be secured to bus bar 15 by compressing tubular bus bar 15 around insert 55 and further by stitching 145 that couples bus bar 15 to heating element 10.

Figure 2D further illustrates lead 205 coupled to insert 55, for example, via soldering, and an insulating tube and strain relief 54, for example, a polymer shrink tube, surrounding the coupling between lead 205 and insert 55.

Returning now to Figure 2B, temperature sensor assembly 421 will be described in greater detail. Figure 2B illustrates assembly 421 including a substrate 211, for example, of polyimide (Kapton), on which a temperature sensor 21, for example, a surface mount chip thermistor (such as a Panasonic ERT-J1VG103FA: 10K, 1% chip thermistor), is mounted; a heat spreader 212, for example, a copper or aluminum foil, is mounted to an opposite side of substrate 211, for example, being bonded with a pressure sensitive adhesive; substrate 211 is relatively thin, for example about 0.0005 inch thick, so that heat transfer between heat spreader 212 and sensor is not significantly impeded. Temperature sensor assembly 421 may be bonded to layer 210 with an adhesive layer 213, for example, hotmelt EVA. Although not shown, it should be noted that sensor assembly 421 may be potted with a flexible electrically insulating material, such as silicon or polyurethane.

According to the illustrated embodiment, heat spreader 212 is sized to contact an enlarged surface area so that a temperature sensed by sensor 21 is more representative of an average temperature over a region of heating element 10 surrounding sensor 21, which is positioned such that, when a heating blanket including heating element 10 is placed over a body, the regions surrounding sensor 21 will be in conductive contact with the body. As previously described, it is desirable that a temperature of approximately 43°C be maintained over a surface of heating element 10 which is in conductive contact with a body of a patient undergoing surgery. Other types of heat spreaders, in addition to metallic foils, include metallic meshes or screens, or an adhesive/epoxy filled with a thermally conductive material.

Heat spreader 212 is a desirable component of a temperature sensor assembly, according to some embodiments of the present invention, since conductive fabrics employed by heating element 10, such as those previously described, may not exhibit uniform resistance across surface areas thereof. Heat spreader 212, having a surface area that does not exceed approximately four square inches, according to a preferred embodiment, may be effective in averaging out relatively small scale spatial resistance variation, for example, about 3% to 10% variability over less than about one or two inches. Such a limitation on heat spreader 212 surface area may be necessary so that heat spreader 212 does not become too bulky, since the larger the surface area, the greater the thickness of spreader 212 needed in order to maintain effective heat transfer across spreader 212 and to sensor 21. In addition, if spreader 212 is too thick, a thermal mass of spreader 212 will cause spreader 212 to respond too slowly to changes in heat loss or gain by heating element. According to an exemplary embodiment of the present invention, spreader 212 has a surface area of no greater than approximately four square inches and a thickness of no greater than approximately 0.001 inch. Some alternate embodiments of the present invention address a non-uniform resistance across a surface area of element 10 by employing a distributed temperature sensor, for example, a resistance temperature detector (RTD) laid out in flat plane across a surface of heating element 10, or by employing an infrared temperature measurement device positioned to receive thermal radiation from a given area of heating element 10. An additional alternate embodiment is contemplated in which an array of temperature sensors are positioned over the surface of heating element 10, being spaced apart so as to collect temperature readings which may be averaged to account for resistance variance.

According to a preferred embodiment, assembly 421 includes a second, redundant temperature sensor mounted to substrate 211, close enough to sensor 21 to detect approximately the same temperature; while sensor 21 may be coupled to a microprocessor temperature control, the second sensor, for example, a chip thermistor similar to sensor 21, may be coupled to an analog over-temperature cutout that cuts power to element 10, and/or sends a signal triggering an audible or visible alarm. The design of the second sensor may be the same as the first sensor and need not be described again. Another safety check may be provided by mounting an identification resistor to substrate 211 in order to detect an increase in resistance of element 10, due, for example, to degradation of the material of element 10, or a fractured bus bar; the optional identification resistor monitors resistance of heating element 10 and compares the measured resistance to an original resistance of element 10.

According to some embodiments of the present invention, for example as illustrated in Figure 2A, super over-temperature sensors 41 are incorporated to detect overheating of areas of assembly 250 susceptible to rucking, that is areas, for example, lateral portions 11, 12, where assembly 250 is most likely to be folded over on itself, either inadvertently or on purpose to gain access to a portion of a patient disposed beneath a blanket including assembly 250. An area of assembly 250 which is beneath the folded-over portion of assembly 250, and not in close proximity to sensor assembly 421, can become significantly warmer due to the additional thermal insulation provided by the folded-over portion that goes undetected by sensor 21. According to preferred embodiments, sensors 41 are wired in series, as illustrated in Figure 2A. Super over-temperature sensors 41 may be set to open, or significantly increase resistance in, a circuit, for example, the over-temperature circuit, thereby activating an alarm and/or cutting power to heating element 10, at prescribed temperatures that are significantly above the normal operating range, for example, temperatures between approximately 45°C and approximately 60°C. Alternately, sensors 41 may be part of the bus bar power circuit, in which case sensors 41 directly shut down power to heating element 10 when in an open condition or add sufficient resistance when in a high resistance condition to substantially reduce heating of element 10.

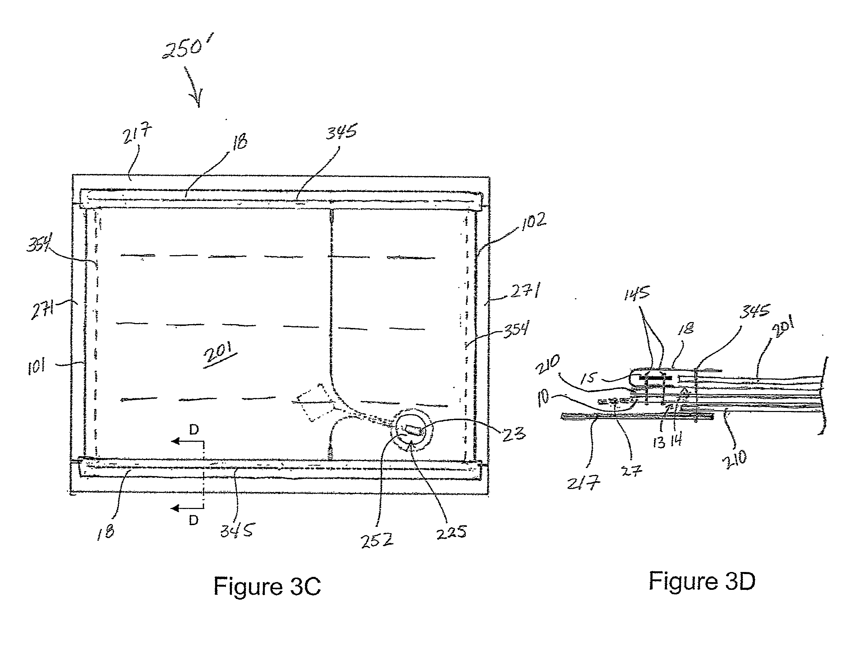

Figure 3A is a top plan view, including partial cut-away views, of a lower body heating blanket 200, according to some embodiments of the present invention, which may be used to keep a patient warm during surgery. Figure 3A illustrates blanket 200 including heating element assembly 250 covered by flexible shell 20; shell 20 protects and isolates assembly 250 from an external environment of blanket 200 and may further protect a patient disposed beneath blanket 200 from electrical shock hazards. According to preferred embodiments of the present invention, shell 20 is waterproof to prevent fluids, for example, bodily fluids, IV fluids, or cleaning fluids, from contacting assembly 250, and may further include an anti-microbial element, for example, being a SILVERion™ antimicrobial fabric available from Domestic Fabrics Corporation. According to the illustrated embodiment, blanket 200 further includes a layer of thermal insulation 201 extending over a top side (corresponding to side 14 of heating element 10) of assembly 250; layer 201 may or may not be bonded to a surface of assembly 250. Layer 201 may serve to prevent heat loss away from a body disposed on the opposite side of blanket 200, particularly if a heat sink comes into contact with the top side of blanket 200. Figure 3C illustrates insulation 201 extending over an entire surface of side 14 of heating element 10 and over sensor assembly 421. According to the illustrated embodiment, layer 201 is secured to heating element assembly 250 to form an assembly 250', as will be described in greater detail below. According to an exemplary embodiment of the present invention, insulating layer 201 comprises a polymer foam, for example, a 1 pound density 30 ILD urethane foam, which has a thickness between approximately 1/8th inch and approximately 3/4th inch. According to alternate embodiments layer 201 comprises any, or a combination of the following: high loft fibrous polymeric non-woven material, non-woven cellulose material, and air, for example, held within a polymeric film bubble.

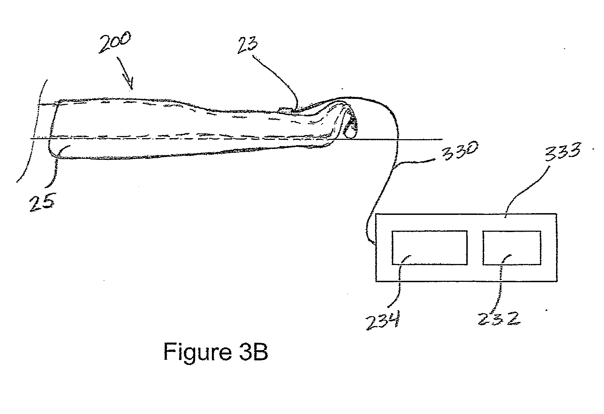

Figure 3A further illustrates shell 20 forming flaps 25 extending laterally from either side of assembly 250 and a foot flap or drape 26 extending longitudinally from assembly 250. According to exemplary embodiments of the present invention, a length of assembly 250 is either approximately 28 inches or approximately 48 inches, the shorter length providing adequate coverage for smaller patients or a smaller portion of an average adult patient. Figure 3B is a schematic side view of blanket 200 draped over a lower body portion of a patient. With reference to Figure 3B it may be appreciated that flaps 25, extending down on either side of the patient, and foot drape 26, being folded under and secured by reversible fasteners 29 (Figure 3A) to form a pocket about the feet of the patient, together effectively enclose the lower body portion of the patient to prevent heat loss. Fasteners 29 may be any suitable type, for example, hook-and-loop or snap. With further reference to Figure 3B, it may also be appreciated that neither shell 20 nor insulation layer 201 add appreciable stiffness to heating element 10 so that blanket 200 conforms nicely to the contour of the patient's lower body. With reference to Figure 2A, in conjunction with Figure 3B, it may be appreciated that temperature sensor assembly 421 is located on assembly 250 so that, when blanket 200 including assembly 250 is draped over the lower body of the patient, the area of heating element 10 surrounding sensor assembly 421 will be in conductive contact with one of the legs of the patient in order to maintain a safe temperature distribution across element 10.

According to some embodiments of the present invention, shell 20 includes top and bottom sheets extending over either side of assembly 250; the two sheets of shell 20 are coupled together along a seal zone 22 (shown with cross-hatching in the cut-away portion of Figure 3A) that extends about a perimeter edge 2000 of blanket 200, and within perimeter edge 2000 to form zones, or pockets, where a gap exists between the two sheets. According to an exemplary embodiment of the present invention, shell 20 comprises a nylon fabric having an overlay of polyurethane coating to provide waterproofing; the coating is on at least an inner surface of each of the two sheets, further facilitating a heat seal between the two sheets, for example, along seal zone 22, according to preferred embodiments. It should be noted that, according to alternate embodiments of the present invention, a covering for heating assemblies, such as heating assembly 250, may be removable and, thus, include a reversible closure facilitating removal of a heating assembly therefrom and insertion of the same or another heating assembly therein.

Figure 3A further illustrates flaps 25 including zones where there are gaps between the sheets to enclose weighting members, which are shown as relatively flat plastic slabs 255. Alternately flaps 25 can be weighted by attaching weighting members to exterior surfaces thereof. Examples of other suitable weighting members include but are not limited to a metal chain, a metal spring, lead shot, plastic rods and sand. The weighting of flaps 25 causes flaps 25 to hang down in order to provide a more secure air seal about the patient. The weighting members may further discourage a clinician from tucking flaps 25 under the patient as a safety feature to help to prevent a portion of the blanket containing heating element 10 from coming into relatively high pressure contact with the patient, where it could cause serious burns; as such, the weighting members are relatively stiff and/or form a lump at the outer edge of flaps 25. Relatively stiff flap weighting members 255, for example, batten-like flat plastic slabs 255, by extending along the length of assembly 250, may further prevent inadvertent rucking of blanket 200, that is, the folding of blanket 200 over on itself which could lead to over-heating of a portion of heating element 10, as previously described. However, with reference to Figure 3A, seal zone 22 extending between members 205 along each flap 25 can predetermine a folding location; the predetermined folding location can prevent overheating (due to the location of sensor assembly 421) or can dictate the placement of super over-temperature sensors 41, as previously described.

Figure 3C is a top plan view, including partial cut-away views, of heating element assembly 250', which may be incorporated in blanket 200; and Figure 3D is a cross-section view through section line D-D of Figure 3C. Figures 3C-D illustrates heating element assembly 250' including heating element 10 overlaid with electrical insulation 210 on both sides 13, 14 and thermal insulation layer 201 extending over the top side 14 thereof (dashed lines show leads and sensor assembly beneath layer 201). According to the illustrated embodiment, layer 201 is inserted beneath a portion of each insulating member 18, each which has been folded over the respective bus bar 15, for example as illustrated by arrow B in Figure 1C, and then held in place by a respective row of non-conductive stitching 345 that extends through member 18, layer 201 and heating element 10. Although not shown, it should be appreciated that layer 201 may further extend over bus bars 15. Although layer 210 is shown extending beneath layer 201 on side 14 of heating element, according to alternate embodiments, layer 201 independently performs as a thermal and electrical insulation so that layer 210 is not required on side 14 of heating element 10. Figure 3C further illustrates, with longitudinally extending dashed lines, a plurality of optional slits in layer 201, which may extend partially or completely through layer 201, in order to increase the flexibility of assembly 250'. Such slits are desirable if a thickness of layer 201 is such that it prevents blanket 200 from draping effectively about a patient; the optional slits are preferably formed, for example, extending only partially through layer 201 starting from an upper surface thereof, to allow bending of blanket 200 about a patient and to prevent bending of blanket 200 in the opposition direction.

Returning now to Figure 2A, to be referenced in conjunction with Figures 3A-C, connector housing 225 and connector 23 will be described in greater detail. According to certain embodiments, housing 225 is an injection molded thermoplastic, for example, PVC, and may be coupled to assembly 250 by being stitched into place, over insulating layer 210. Figure 2A shows housing 225 including a flange 253 through which such stitching can extend. With reference to Figures 3A-B, it can be seen that connector 23 protrudes from shell 20 of blanket 200 so that an extension cable 330 may couple bus bars 15 to a power source 234, and temperature sensor assembly 421 to a temperature controller 232, both shown incorporated into a console 333. In certain embodiments, power source 234 supplies a pulse-width-modulated voltage to bus bars 15. The controller 232 may function to interrupt such power supply (e.g., in an over-temperature condition) or to modify the duty cycle to control the heating element temperature. According to the illustrated embodiment, a surface 252 of flange 253 of housing 225 protrudes through a hole formed in thermal insulating layer 201 (Figure 3C) so that a seal 202 (Figure 3A) may be formed, for example, by adhesive bonding and/or heat sealing, between an inner surface of shell 20 and surface 252. According to an exemplary embodiment, wherein housing 225 is injection molded PVC and the inner surface of shell 20 is coated with polyurethane, a liquid adhesive, which bonds and heat seals to both PVC and polyurethane, is applied to surface 252 and allowed to cure prior to heat sealing shell 20 to surface 252. With further reference to Figure 3B, it may be appreciated that the location of plug 23 is suitable to keep connector cord 330 well away from the surgical field.

Figures 3C-D further illustrate a pair of securing strips 217, each extending laterally from and alongside respective lateral portions 11, 12 of heating element 10 and each coupled to side 13 of heating element 10 by the respective row of stitching 345. Another pair of securing strips 271 is shown in Figure 3C, each strip 271 extending longitudinally from and alongside respective ends 101, 102 of heating element 10 and being coupled thereto by a respective row of non-conductive stitching 354. Strips 271 may extend over layer 201 or beneath heating element 10. Strips 217 preferably extend over conductive stitching 145 on side 13 of heating element 10, as shown, to provide a layer of insulation that can prevent shorting between portions of side 13 of heating element 10 if element 10 were to fold over on itself along rows of conductive stitching 145 that couple bus bars 15 to heating element 10; however, strips 217 may alternately extend over insulating member 18 on the opposite side of heating element 10. According to the illustrated embodiment, securing strips 217 and 271 are made of a polymer material, for example polyurethane, so that they may be heat sealed between the sheets of shell 20 in corresponding areas of heat seal zone 22 in order to secure heating element assembly 250' within the corresponding gap between the two sheets of shell 20 (Figure 3A). According to an alternate embodiment, for example, shown by dashed lines in Figures 1A and 3D, heating element 10 extends laterally out from each bus bar 15 to a securing edge 27, which may include one or more slots or holes 207 extending therethrough so that inner surfaces of sheets of shell 20 can contact one another to be sealed together and thereby hold edges 27.

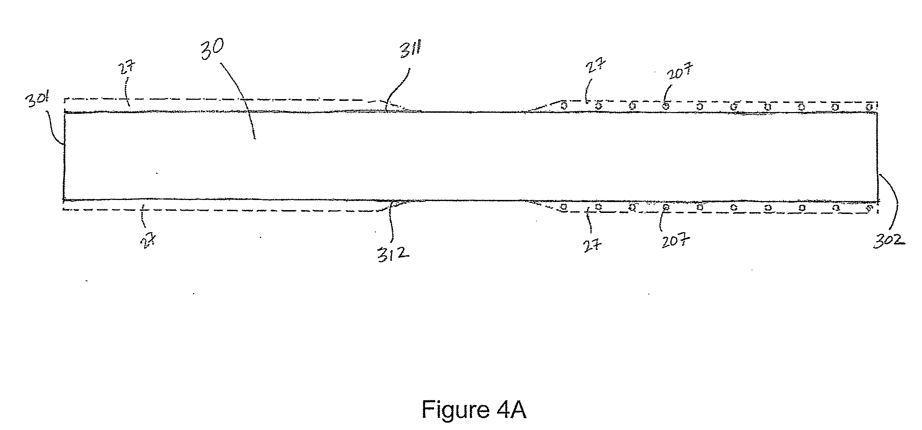

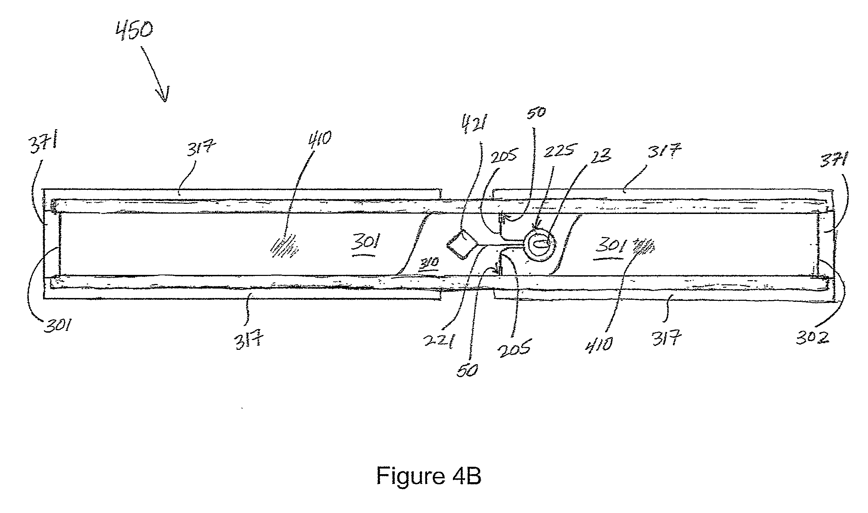

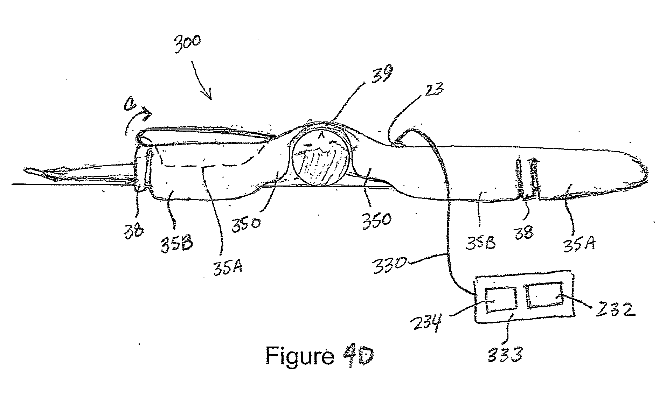

Figure 4A is a plan view of flexible heating element 30, according to some alternate embodiments of the present invention. Heating element 30 is similar in nature to previously described embodiments of heating element 10, being comprised of a conductive fabric, or a fabric incorporating closely spaced conductive elements, for a substantially uniform watt density output, preferably less than approximately 0.5 watts/sq. inch. While a shape of the surface area of heating element 10 is suited for a lower body blanket, such as blanket 200, that would cover a lower abdomen and legs of a patient (Figure 3B) undergoing upper body surgery, the shape of a surface area of heating element 30 is suited for an upper body heating blanket, for example, blanket 300 shown in Figure 4C, that would cover outstretched arms and a chest area of a patient undergoing lower body surgery (Figure 4D). According to an exemplary embodiment for an adult upper body heating blanket, a distance between a first end 301 of element 30 and a second end 302 of element 30 is between about 70 and 80 inches, while a distance between a first lateral edge 311 and a second lateral edge 312 is about 7 to 10 inches. With reference to Figure 4B, which shows heating element 30 incorporated into a heating element assembly 450, it can be seen that bus bars 15 are coupled to element 30 alongside respective lateral edges 311, 312 (Figure 4A). For the narrower spacing between bus bars 15, compared with that for heating element 10 incorporated in blanket 200, element 30, in order to have the desired watt density output, should be comprised of a conductive fabric having a higher resistance than the examples previously recited for heating element 10, for example, on the order of 100 ohms per square, measured with a four point probe. An example of a conductive fabric meeting this resistance requirement is a woven silk-like polyester, for example, known as Pongee, being 100% coated with polypyrrole.

Figure 4B is a top plan view, including partial cut-away views, of heating element assembly 450, according to some embodiments of the present invention, which may be incorporated in blanket 300 shown in Figure 4C. Figure 4B illustrates assembly 450 having a configuration similar to that of assembly 250', which is illustrated in Figures 3C-D. According to the embodiment illustrated in Figure 4B, temperature sensor assembly 421 is coupled to heating element 30 at a location where element 30, when incorporated in an upper body heating blanket, for example, blanket 300, would come into conductive contact with the chest of a patient, for example as illustrated in Figure 4D, in order to maintain a safe temperature distribution across element 30; bus bar junctions 50 and connector housing 225 are located in proximity to sensor assembly 421 in order to keep a length of leads 205 and 221 to a minimum. With reference back to Figures 3C-D, in conjunction with Figure 4B, an electrical insulating layer 310 of assembly 450 corresponds to insulating layers 210 of assembly 250', a thermal insulating layer 301 of assembly 450 corresponds to layer 201of assembly 250', and securing strips 317 and 371 of assembly 450 generally correspond to strips 217 and 271, respectively, of assembly 250'.

Figure 4C is a top plan view, including partial cut-away views, of upper body heating blanket 300, according to some embodiments of the present invention. Figure 4C illustrates blanket 300 including heating element assembly 450 covered by a flexible shell 40; shell 40 protects and isolates assembly 450 from an external environment of blanket 300 and may further protect a patient disposed beneath blanket 300 from electrical shock hazards. According to preferred embodiments, shell 40 is similar to shell 20 of blanket 200 in that shell 40 is relatively durable and waterproof and may further include an antimicrobial element or layer extending over an exterior surface thereof. According to the illustrated embodiment, shell 40, like shell 20, includes top and bottom sheets; the sheets extend over either side of assembly 450 and are coupled together along a seal zone 32 that extends around a perimeter edge 4000 and within edge 4000 to form various zones, or pockets, where gaps exist between the two sheets. The sheets of shell 40 may be heat sealed together along zone 32, as previously described for the sheets of shell 20. With reference to Figure 4B, securing strips 317 may be heat sealed between the sheets of shell 40 in corresponding areas of seal zone 32, on either side of a central narrowed portion 39 of blanket 300, in order to secure heating element assembly 450 within the corresponding gap between the two sheets of shell 40. According to an alternate embodiment, for example, as shown with dashed lines in Figure 4A, lateral edges 311, 312 of heating element 30 extend out to form securing edges 27 that each include slots or holes 207 extending therethrough so that inner surfaces of sheets of shell 40 can contact one another to be sealed together and thereby hold edges 27. It should be noted that either of blankets 200, 300, according to alternate embodiments of the present invention, may include more than one heating element 10, 30 and more than one assembly 250/250', 450.

With reference to Figure 4C, it may be appreciated that blanket 300 is symmetrical about a central axis 30 and about another central axis, which is orthogonal to axis 30. Figure 4C illustrates shell 40 forming flaps 35A, 35B and 350, each of which having a mirrored counterpart across central axis 30 and across the central axis orthogonal to axis 30. According to the illustrated embodiment, each of flaps 35A, B are weighted in a fashion similar to that described for flaps 25 of blanket 200 include weighting members 305, which are similar to members 255 of blanket 200, and which may stiffen flaps 35A, B (dashed lines indicate outlines of members 305 held between the sheets of cover 40 by surrounding areas of seal zone 32), or in any of the alternative fashions previously described.

Figure 4C further illustrates straps 38, each extending between respective flaps 35A-B. With reference to Figure 4D, which is a schematic end view of blanket 300 draped over an upper body portion of a patient, it may be appreciated that flaps 35A-B and 350 extend downward to enclose the outstretched arms of the patient in order to prevent heat loss and that straps 38 secure blanket 300 about the patient. Opposing straps 38 may be secured together with reversible fasteners, examples of which include, without limitation, magnetic fasteners, either embedded within straps 38 or coupled to outer surfaces thereof, mating hook-and-loop fasteners, attached to opposing straps 38, and mating snap fasteners, attached to opposing straps. According to preferred embodiments, portions of perimeter edge 4000 defining narrowed portion 39, which extends across a chest of the patient, are either rounded or padded to provide a softer interface with the patient's chin if blanket 300 were to slip off the patient's chest toward the patient's chin.

With further reference to Figure 4D, it may also be appreciated that, when blanket 300 is positioned over the patient, each strap 38 is positioned in proximity to an elbow of the patient so that either end portion of blanket 300, corresponding to each pair of flaps 35A, may be temporarily folded back, as illustrated, per arrow C, in order for a clinician to access the patient's arm, for example, to insert or adjust an IV. According to some embodiments of the present invention, super over-temperature sensors, for example, sensors 41, previously described, are included in blanket 300 being located according to the anticipated folds, for example at general locations 410 illustrated in Figures 4B-C, in order to detect over-heating, which may occur if blanket 300 is folded over on itself, as illustrated in Figure 4D, for too long a time, and, particularly, if flaps 35A of folded-back portion of blanket are allowed to extend downward as illustrated with the dashed line in Figure 4D. Figure 4D further illustrates connector cord 330 plugged into connector 23 to couple heating element 30 and temperature sensor assembly 421 of blanket 300 to control console 333.

In the foregoing detailed description, the invention has been described with reference to specific embodiments. However, it may be appreciated that various modifications and changes can be made without departing from the scope of the invention as set forth in the appended claims. Although embodiments of the invention are described in the context of a hospital operating room, it is contemplated that some embodiments of the invention may be used in other environments.

**Claims**

1. An electric warming blanket (100; 200), comprising:

   a flexible conductive fabric heater (10) including a first side (13) and a second side (14);
   a first layer of water resistant material (20) disposed over the first side of the heater, being un-adhered thereto, and forming an outer surface of the blanket when the blanket is draped over an object or a person to be warmed; and
   a second layer of water resistant material (20) disposed over the second side of the heater, being un-adhered thereto, and forming an inner surface of the blanket, adjacent to the object or the person, when the blanket is draped thereover, the first layer of water resistant material coupled to the second layer of water resistant material about a perimeter of the heater to form a substantially hermetically sealed space for the heater;
   a layer of thermal insulation (201) disposed between the first side of the heater and the first layer of water resistant material;

a temperature controller (232);
a temperature sensor assembly (421) coupled to the heater and the controller; and
an electric power source (234) coupled to the heater and to the temperature controller, **characterized in that**:

   the flexible conductive fabric heater has at least one of the first and second sides having a surface area and a substantially uniform watt density output across the surface area when the fabric heater is electrically powered;
   the temperature sensor assembly comprises a temperature sensor (21; 121), the temperature sensor providing input of a temperature over a portion of the surface area of the heater surrounding the temperature sensor to the temperature controller; and
   the power source is controlled to provide the substantially uniform watt density output for the heater according to a temperature sensed by the temperature sensor such that when a first portion of the heater including the temperature sensor is in conductive contact with the body and a second portion is not in conductive contact with the body, the second portion has a higher equilibrium temperature than the first portion.

2. The blanket (100; 200) of claim 1, further comprising:

   a layer of thermal insulation disposed between the temperature sensor assembly and the first layer of water resistant material.

3. The blanket (100; 200) of claim 1, wherein:

   the temperature sensor assembly (421) includes a heat spreader (212), the heat spreader comprising a metal foil disposed between the temperature sensor (21; 121) and the heater (10), a portion being disposed along the surface area so as to be in conductive contact with the patient when the blanket is place over the patient.

4. The blanket (100; 200) of claim 3, further comprising:

   a second temperature sensor mounted proximate to the temperature sensor (21; 121), the second temperature sensor for detecting approximately the same heater temperature as the temperature sensor.

5. The blanket (100; 200) of claim 1, further comprising:

   a first layer (210) of non-conductive flexible porous material bonded to the first side of the heater (10); and

a second layer (210) of non-conductive flexible porous material bonded to the second side of the heater.

6. The blanket (100; 200) of claim 1, further comprising:

at least one first stiffening member supported by the first (20) and second (20) layers of water resistant material; and

at least one second stiffening member supported by the first and second layers of water resistant material;

the heater (10) including a first lateral edge (11) and a second lateral edge (12) opposite the first lateral edge, the at least one first stiffening member extending beyond the first lateral edge of the heater and the at least one second stiffening member extending beyond the second lateral edge of the heater.

7. The blanket (100; 200) of claim 1, further comprising:

a first conductive bus bar (15) coupled to the heater (10) and extending alongside a first lateral edge (11) of the heater;

a second conductive bus bar (15) coupled to the heater and extending alongside a second lateral edge (12) of the heater, the second lateral edge being opposite the first lateral edge;

the temperature sensor (21; 121) coupled to the heater at a location between the first and second bus bars, where the heater will be in conductive contact with a body when the blanket is draped over the body; the temperature sensor providing input to the temperature controller (232), the controller adapted to control a supply of power to the first and second bus bars, the supply of power being based on a sensed temperature from the temperature sensor; and

at least one super over-temperature sensor (41) coupled to the heater between the first and second bus bars, the at least one super over-temperature sensor adapted to interrupt the supply of power to the first and second bus bars when a temperature sensed by the at least one super over-temperature sensor exceeds a prescribed temperature.

8. The blanket (100; 200) of claim 7, wherein:

the at least one super over-temperature sensor (41) comprises a plurality of super over-temperature sensors wired in series with one another and each exhibiting a significant resistance increase at prescribed temperatures above a normal operating range of the heater.

9. The blanket (100; 200) of claim 1, further comprising:

a first conductive bus bar (15) disposed alongside a first lateral edge (11) of the heater (10);

a second conductive bus bar (15) disposed alongside a second lateral edge (12) of the heater, the second lateral edge being opposite to the first lateral edge;

a first row of stitching (145) connecting the first bus bar to the heater;

a second row of stitching (145) connecting the second bus bar to the heater;

the first and second rows of stitching each comprising electrically conductive thread;

the first and second bus bars adapted for coupling to the (234) power source for powering the heater;

a first electrically insulating member (18) interposed between the first bus bar and the heater and being secured therebetween by the first row of stitching;

a second electrically insulating member (18) interposed between the second bus bar and the heater and being secured therebetween by the second row of stitching; and

the first and second insulating members preventing direct electrical contact between respective first and second bus bars and the heater.

10. The blanket (100; 200) of claim 1, further comprising:

a first conductive bus bar (15) disposed alongside a first edge (11) of the heater (10) and extending beyond first (101) and second (102) ends of the heater to be terminated just beyond either end, the first edge extending between the first and second ends of the heater;

a second conductive bus bar (15) disposed alongside a second edge (12) of the heater and extending beyond the first and second ends of the heater to be terminated just beyond either end, the second edge being opposite the first edge and extending between the first and second ends of the heater;

at least one first row of stitching (145) coupling the first bus bar to the heater, the at least one first row of stitching extending along the first bar;

at least one second row of stitching (145) coupling the second bus bar to the heater, the at least one second row of stitching extending along the second bus bar; and

the first and second bus bars adapted for coupling to the power source (234) for powering the heater of the conductive fabric heater.

11. The blanket (100; 200) of claim 1, further comprising:

a conductive first bus bar (15) having a first side disposed against the heater (10), the bus bar extending alongside a first edge (11) of the heater and having the first edge of the heater folded thereover to be disposed against a second side of the first bus bar;

a conductive second bus bar (15) having a first side disposed against the heater, the bus bar extending alongside a second edge (12) of the heater, opposite the first edge, and having the second edge of the heater folded thereover to be disposed against a second side of the second bus bar;

at least one first row of stitching (145) coupling the first bus bar to the heater by extending through the folded over first edge of the heater, the first bus bar, and the heater disposed against the first side of the first bus bar;

at least one second row of stitching (145) coupling the second bus bar to the heater by extending through the folded over second edge of the heater, the second bus bar, and the heater disposed against the first side of the second bus bar; and

the first and second bus bars adapted for coupling to the power source (234) for powering the conductive fabric heater.

12. The blanket (100; 200) of claim 1, further comprising:

a first conductive bus bar (15) disposed alongside a first edge (11) of the heater (10);

a second conductive bus bar (15) disposed alongside a second edge (12) of the heater, opposite the first edge;

at least one first row of stitching (145) coupling the first bus bar to the heater, the at least one first row of stitching extending along the first bar;

a first ribbon of conductive material interposed between the first bus bar and the heater;

at least one second row of stitching (145) coupling the second bus bar to the heater, the at least one second row of stitching extending along the second bus bar; and

a second ribbon of conductive material interposed between the second bus bar and the heater;

the first and second bus bars adapted for coupling to the power source (234) for powering the conductive fabric heater.

13. The blanket (100; 200) of claim 1, further comprising:

a conductive first bus bar (15) disposed alongside a first edge (11) of the heater (10);

a conductive second bus bar (15) disposed alongside a second edge (12) of the heater, opposite the first edge;

at least two first rows of stitching (145) coupling the first bus bar to the heater, the at least two first rows of stitching extending along the first bus bar and comprising electrically conductive thread; and

at least two second rows of stitching (145) coupling the second bus bar to the heater, the at least two second rows of stitching extending along the second bus bar and comprising electrically conductive thread, and

the first and second bus bars adapted for coupling to the power source (234) for powering the heater.

14. The blanket (100; 200) of claim 1, wherein:

the heater (10) comprises one of the following: carbon, or a non-conductive material coated with a conductive material such as carbon, polypyrrole, metallic ink, or carbonized ink.

**Patentansprüche**

1. Elektrische Wärmedecke (100; 200), umfassend:

eine flexible leitfähige Stoffheizvorrichtung (10) mit einer ersten Seite (13) und einer zweiten Seite (14);

eine erste Schicht aus wasserabstoßendem Material (20), die über der ersten Seite der Heizvorrichtung angeordnet und nicht daran gehaftet ist und die eine äußere Oberfläche der Decke bildet, wenn die Decke über ein Objekt oder eine Person, das bzw. die zu wärmen ist, gelegt wird; und

eine zweite Schicht aus wasserabstoßendem Material (20), die über der zweiten Seite der Heizvorrichtung angeordnet und nicht daran gehaftet ist und eine innere Oberfläche der Decke angrenzend an das Objekt oder die Person bildet, wenn die Decke darüber gelegt wird, wobei die erste Schicht aus wasserabstoßendem Material über einem Umfang der Heizvorrichtung mit der zweiten Schicht aus wasserabstoßendem Material gekoppelt ist, um einen im Wesentlichen hermetisch abgedichteten Raum für die Heizvorrichtung zu bilden;

eine Schicht aus Wärmeisolierung (201), die zwischen der ersten Seite der Heizvorrichtung und der ersten Schicht aus wasserabstoßendem Material angeordnet ist;

einen Temperaturregler (232);

eine mit der Heizvorrichtung und dem Regler gekoppelte Temperatursensorbaugruppe (421); und

eine mit der Heizvorrichtung und mit dem Temperaturregler gekoppelte elektrische Stromquelle (234),

**dadurch gekennzeichnet, dass**

von der flexiblen leitfähigen Stoffheizvorrichtung mindestens eine der ersten und zweiten Seite einen Flächeninhalt und eine im Wesentlichen gleichförmige über den Flächeninhalt ausgegebene Leistungsdichte aufweist, wenn die Stoffheizvorrichtung elektrisch mit Strom versorgt wird;

die Temperatursensorbaugruppe einen Temperatursensor (21; 121) umfasst, wobei der Temperatursensor Eingabe einer Temperatur über einen Teil des Flächeninhalts der Heizvorrichtung, der den Temperatursensor umgibt, in den Temperaturregler bereitstellt; und

die Stromquelle geregelt wird, um die im Wesentlichen gleichförmige Leistungsdichteausgabe für die Heizvorrichtung gemäß einer durch den Temperatursensor erfassten Temperatur dergestalt bereitzustellen, dass, wenn sich ein erster Teil der Heizvorrichtung, der den Temperatursensor enthält, in leitfähigem Kontakt mit dem Körper befindet und sich ein zweiter Teil nicht in leitfähigem Kontakt mit dem Körper befindet, der zweite Teil eine höhere Gleichgewichtstemperatur als der erste Teil aufweist.

2. Decke (100; 200) nach Anspruch 1, ferner umfassend:

eine Schicht aus Wärmeisolierung, die zwischen der Temperatursensorbaugruppe und der ersten Schicht aus wasserabstoßendem Material angeordnet ist.

3. Decke (100; 200) nach Anspruch 1, wobei die Temperatursensorbaugruppe (421) einen Wärmeverteiler (212) umfasst, wobei der Wärmeverteiler eine zwischen dem Temperatursensor (21; 121) und der Heizvorrichtung (10) angeordnete Metallfolie umfasst, wobei ein Teil entlang dem Flächeninhalt angeordnet ist, um sich so in leitfähigem Kontakt mit dem Patienten zu befinden, wenn die Decke über dem Patienten angeordnet wird.

4. Decke (100; 200) nach Anspruch 3, ferner umfassend:

einen in der Nähe des Temperatursensors (21; 121) angebrachten zweiten Temperatursensor, wobei der zweite Temperatursensor zum Detektieren ungefähr derselben Heizvorrichtungstemperatur wie der Temperatursensor dient.

5. Decke (100; 200) nach Anspruch 1, ferner umfassend:

eine erste Schicht (210) aus nichtleitfähigem flexiblem porösem Material, das an die erste Seite der Heizvorrichtung (10) gebondet ist; und eine zweite Schicht (210) aus nichtleitfähigem flexiblem porösem Material, das an die zweite Seite der Heizvorrichtung gebondet ist.

6. Decke (100; 200) nach Anspruch 1, ferner umfassend:

mindestens ein durch die erste (20) und zweite (20) Schicht aus wasserabstoßendem Material gehaltenes erstes Versteifungsglied; und mindestens ein durch die erste und zweite Schicht aus wasserabstoßendem Material gehaltenes zweites Versteifungsglied; wobei die Heizvorrichtung (10) einen ersten seitlichen Rand (11) und einen zweiten seitlichen Rand (12) gegenüber dem ersten seitlichen Rand umfasst, wobei sich das mindestens eine erste Versteifungsglied über den ersten seitlichen Rand der Heizvorrichtung hinaus erstreckt und sich das mindestens eine zweite Versteifungsglied über den zweiten seitlichen Rand der Heizvorrichtung hinaus erstreckt.

7. Decke (100; 200) nach Anspruch 1, ferner umfassend:

eine erste leitfähige Sammelschiene (15), die mit der Heizvorrichtung (10) gekoppelt ist und sich entlang eines ersten seitlichen Rands (11) der Heizvorrichtung erstreckt; eine zweite leitfähige Sammelschiene (15), die mit der Heizvorrichtung gekoppelt ist und sich entlang eines zweiten seitlichen Rands (12) der Heizvorrichtung erstreckt, wobei der zweite seitliche Rand dem ersten seitlichen Rand gegenüber ist; einen Temperatursensor (21; 121), der an einem Ort zwischen der ersten und zweiten Sammelschiene mit der Heizvorrichtung gekoppelt ist, wobei sich die Heizvorrichtung in leitfähigem Kontakt mit einem Körper befinden wird, wenn die Decke über den Körper gelegt wird; wobei der Temperatursensor dem Temperaturregler (232) Eingaben zuführt, der Regler dafür ausgelegt ist, die Versorgung der ersten und zweiten Sammelschiene mit Strom zu regeln und die Versorgung mit Strom auf einer erfassten Temperatur aus dem Temperatursensor basiert; und mindestens einen superlativen Übertemperatursensor (41), der zwischen der ersten und zweiten Sammelschiene mit der Heizvorrichtung gekoppelt ist, wobei der mindestens eine superlative Übertemperatursensor dafür ausgelegt ist, die Versorgung der ersten und zweiten Sammelschiene mit Strom zu unterbrechen, wenn eine durch den mindestens einen superlativen Übertemperatursensor erfasste Temperatur eine vorgeschriebene Temperatur überschreitet.

8. Decke (100; 200) nach Anspruch 7, wobei der mindestens eine superlative Übertemperatursensor (41) mehrere miteinander in Reihe geschaltete superlative Übertemperatursensoren umfasst und jeder bei vorgeschriebenen Temperaturen über einem normalen Betriebsbereich der Heizvorrichtung eine signifikante Widerstandszunahme aufweist.

9. Decke (100; 200) nach Anspruch 1, ferner umfassend:

eine entlang eines ersten seitlichen Rands (11) der Heizvorrichtung (10) angeordnete erste leitfähige Sammelschiene (15);

eine entlang eines zweiten seitlichen Rands (12) der Heizvorrichtung angeordnete zweite leitfähige Sammelschiene (15), wobei der zweite seitliche Rand dem ersten seitlichen Rand gegenüberliegt;

eine erste Reihe von Naht (145), die die erste Sammelschiene mit der Heizvorrichtung verbindet;

eine zweite Reihe von Naht (145), die die zweite Sammelschiene mit der Heizvorrichtung verbindet;

wobei die erste und zweite Reihe von Naht jeweils einen elektrisch leitfähigen Faden umfassen;

wobei die erste und zweite Sammelschiene dafür ausgelegt sind, mit der Stromquelle zur Stromversorgung der Heizvorrichtung gekoppelt zu werden (234);

ein erstes elektrisch isolierendes Glied (18), das zwischen der ersten Sammelschiene und der Heizvorrichtung angeordnet und dazwischen durch die erste Reihe von Naht befestigt ist;

ein zweites elektrisch isolierendes Glied (18), das zwischen der zweiten Sammelschiene und der Heizvorrichtung angeordnet und dazwischen durch die zweite Reihe von Naht befestigt ist; und

wobei das erste und zweite isolierende Glied direkten elektrischen Kontakt zwischen der ersten bzw. zweiten Sammelschiene und der Heizvorrichtung verhindern.

10. Decke (100; 200) nach Anspruch 1, ferner umfassend:

eine erste leitfähige Sammelschiene (15), die entlang eines ersten Rands (11) der Heizvorrichtung (10) angeordnet ist und sich über ein erstes (101) und zweites (102) Ende der Heizvorrichtung hinaus erstreckt, um gerade eben über jedes Ende hinaus abgeschlossen zu werden, wobei sich der erste Rand zwischen dem ersten und zweiten Ende der Heizvorrichtung erstreckt;

eine zweite leitfähige Sammelschiene (15), die entlang eines zweiten Rands (12) der Heizvorrichtung angeordnet ist und sich über das erste und zweite Ende der Heizvorrichtung hinaus erstreckt, um gerade eben über jedes Ende hinaus abgeschlossen zu werden, wobei der zweite Rand dem ersten Rand gegenüberliegt und sich zwischen dem ersten und zweiten Ende der Heizvorrichtung erstreckt;

mindestens eine erste Reihe von Naht (145), die die erste Sammelschiene mit der Heizvorrichtung koppelt, wobei sich die mindestens eine erste Reihe von Naht entlang der ersten Schiene erstreckt;

mindestens eine zweite Reihe von Naht (145), die die zweite Sammelschiene mit der Heizvorrichtung koppelt, wobei sich die mindestens eine zweite Reihe von Naht entlang der zweiten Sammelschiene erstreckt; und

die erste und zweite Sammelschiene, ausgelegt zur Kopplung mit der Stromquelle (234) zur Versorgung der Heizvorrichtung der leitfähigen Stoffheizvorrichtung mit Strom.

11. Decke (100; 200) nach Anspruch 1, ferner umfassend:

eine leitfähige erste Sammelschiene (15) mit einer gegen die Heizvorrichtung (10) angeordneten ersten Seite, wobei sich die Sammelschiene entlang eines ersten Rands (11) der Heizvorrichtung erstreckt und den ersten Rand der Heizvorrichtung darüber gefaltet aufweist, um gegen eine zweite Seite der ersten Sammelschiene angeordnet zu werden;

eine leitfähige zweite Sammelschiene (15) mit einer gegen die Heizvorrichtung angeordneten ersten Seite, wobei sich die Sammelschiene entlang eines zweiten Rands (12) der Heizvorrichtung gegenüber dem ersten Rand erstreckt und den zweiten Rand der Heizvorrichtung darüber gefaltet aufweist, um gegen eine zweite Seite der zweiten Sammelschiene angeordnet zu werden;

mindestens eine erste Reihe von Naht (145), die die erste Sammelschiene mit der Heizvorrichtung koppelt, indem sie sich durch den übergefalteten ersten Rand der Heizvorrichtung, die erste Sammelschiene und die gegen die erste Seite der ersten Sammelschiene angeordnete Heizvorrichtung erstreckt;

mindestens eine zweite Reihe von Naht (145), die die zweite Sammelschiene mit der Heizvorrichtung koppelt, indem sie sich durch den übergefalteten zweiten Rand der Heizvorrichtung, die zweite Sammelschiene und die gegen die erste Seite der zweiten Sammelschiene angeordnete Heizvorrichtung erstreckt; und

die erste und zweite Sammelschiene, ausgelegt zur Kopplung mit der Stromquelle (234) zur Versorgung der leitfähigen Stoffheizvorrichtung mit Strom.

12. Decke (100; 200) nach Anspruch 1, ferner umfassend:

eine erste leitfähige Sammelschiene (15), die entlang eines ersten Rands (11) der Heizvorrichtung (10) angeordnet ist;
eine zweite leitfähige Sammelschiene (15), die entlang eines zweiten Rands (12) der Heizvorrichtung gegenüber dem ersten Rand angeordnet ist;
mindestens eine erste Reihe von Naht (145), die die erste Sammelschiene mit der Heizvorrichtung koppelt, wobei sich die mindestens eine erste Reihe von Naht entlang der ersten Schiene erstreckt;
ein erstes Band aus leitfähigem Material, das zwischen der ersten Sammelschiene und der Heizvorrichtung angeordnet ist;
mindestens eine zweite Reihe von Naht (145), die die zweite Sammelschiene mit der Heizvorrichtung koppelt, wobei sich die mindestens eine Reihe von Naht entlang der zweiten Sammelschiene erstreckt; und
ein zweites Band aus leitfähigem Material, das zwischen der zweiten Sammelschiene und der Heizvorrichtung angeordnet ist;
die erste und zweite Sammelschiene, ausgelegt zur Kopplung mit der Stromquelle (234) zur Versorgung der leitfähigen Stoffheizvorrichtung mit Strom.

13. Decke (100; 200) nach Anspruch 1, ferner umfassend:

eine erste leitfähige Sammelschiene (15), die entlang eines ersten Rands (11) der Heizvorrichtung (10) angeordnet ist;
eine zweite leitfähige Sammelschiene (15), die entlang eines zweiten Rands (12) der Heizvorrichtung gegenüber dem ersten Rand angeordnet ist;
mindestens zwei erste Reihen von Naht (145), die die erste Sammelschiene mit der Heizvorrichtung koppeln, wobei sich die mindestens zwei ersten Reihen von Naht entlang der ersten Sammelschiene erstrecken und einen elektrisch leitfähigen Faden umfassen; und
mindestens zwei zweite Reihen von Naht (145), die die zweite Sammelschiene mit der Heizvorrichtung koppeln, wobei sich die mindestens zwei zweiten Reihen von Naht entlang der zweiten Sammelschiene erstrecken und einen elektrisch leitfähigen Faden umfassen; und
die erste und zweite Sammelschiene, ausgelegt zur Kopplung mit der Stromquelle (234) zur Versorgung der Heizvorrichtung mit Strom.

14. Decke (100; 200) nach Anspruch 1, wobei die Heizvorrichtung (10) Kohlenstoff oder ein mit einem leitfähigen Material wie Kohlenstoff beschichtetes nichtleitfähiges Material, Polypyrrol, Metalltinte oder karbonisierte Tinte umfasst.

**Revendications**

1. Couverture chauffante électrique (100; 200), comprenant:

un dispositif de chauffage de tissu conducteur flexible (10) présentant un premier côté (13) et un deuxième côté (14);
une première couche de matière résistant à l'eau (20) disposée sur le premier côté du dispositif de chauffage, sans adhérer à celui-ci, et formant une surface extérieure de la couverture lorsque la couverture est drapée sur un objet ou une personne à réchauffer; et
une deuxième couche de matière résistant à l'eau (20) disposée sur le deuxième côté du dispositif de chauffage, sans adhérer à celui-ci, et formant une surface intérieure de la couverture, adjacente à l'objet ou à la personne, lorsque la couverture est drapée sur celui-ci/celle-ci, la première couche de matière résistant à l'eau étant couplée à la deuxième couche de matière résistant à l'eau autour d'un périmètre du dispositif de chauffage de manière à former un espace sensiblement hermétiquement clos pour le dispositif de chauffage;
une couche d'isolation thermique (201) disposée entre le premier côté du dispositif de chauffage et la première couche de matière résistant à l'eau;
un dispositif de commande de température (232);
un ensemble de capteur de température (421) couplé au dispositif de chauffage et au dispositif de commande; et
une source de puissance électrique (234) couplée au dispositif de chauffage et au dispositif de commande de température,
**caractérisée en ce que**:

au moins un des premier et deuxième côtés du dispositif de chauffage de tissu conducteur flexible présente une aire de surface et une sortie de densité de watt sensiblement uniforme à travers l'aire de surface lorsque le dispositif de chauffage de tissu est alimenté électriquement;

l'ensemble de capteur de température comprend un capteur de température (21; 121), le capteur de température fournissant une entrée d'une température sur une partie de l'aire de surface du dispositif de chauffage qui entoure le capteur de température au dispositif de commande de température; et la source de puissance est commandée de manière à fournir une sortie de densité de watts sensiblement uniforme pour le dispositif de chauffage selon une température détectée par le capteur de température de telle sorte que lorsqu'une première partie du dispositif de chauffage comprenant le capteur de température se trouve en contact conductif avec le corps et qu'une deuxième partie ne se trouve pas en contact conductif avec le corps, la deuxième partie présente une température d'équilibre plus élevée que la première partie.

2. Couverture (100; 200) selon la revendication 1, comprenant en outre:

une couche d'isolation thermique disposée entre l'ensemble de capteur de température et la première couche de matière résistant à l'eau.

3. Couverture (100; 200) selon la revendication 1, dans laquelle:

l'ensemble de capteur de température (421) comprend un diffuseur de chaleur (212), le diffuseur de chaleur comprenant une feuille de métal disposée entre le capteur de température (21; 121) et le dispositif de chauffage (10), une partie étant disposée le long de l'aire de surface de manière à se trouver en contact conductif avec le patient lorsque la couverture est placée sur le patient.

4. Couverture (100; 200) selon la revendication 3, comprenant en outre:

un deuxième capteur de température monté à proximité du capteur de température (21; 121), le deuxième capteur de température servant à détecter approximativement la même température du dispositif de chauffage que le capteur de température.

5. Couverture (100; 200) selon la revendication 1, comprenant en outre:

une première couche (210) de matière poreuse flexible non conductrice liée au premier côté du dispositif de chauffage (10); et une deuxième couche (210) de matière poreuse flexible non conductrice liée au deuxième côté du dispositif de chauffage.

6. Couverture (100; 200) selon la revendication 1, comprenant en outre:

au moins un premier élément de raidissement supporté par les première (20) et deuxième (20) couches de matière résistant à l'eau, et au moins un deuxième élément de raidissement supporté par les première et deuxième couches de matière résistant à l'eau, le dispositif de chauffage (10) présentant un premier bord latéral (11) et un deuxième bord latéral (12) opposé au premier bord latéral, ledit au moins un premier élément de raidissement s'étendant au-delà du premier bord latéral du dispositif de chauffage, et ledit au moins un deuxième élément de raidissement s'étendant au-delà du deuxième bord latéral du dispositif de chauffage.

7. Couverture (100; 200) selon la revendication 1, comprenant en outre:

une première barre omnibus conductrice (15) couplée au dispositif de chauffage (10) et s'étendant le long d'un premier bord latéral (11) du dispositif de chauffage; une deuxième barre omnibus conductrice (15) couplée au dispositif de chauffage et s'étendant le long d'un deuxième bord latéral (12) du dispositif de chauffage, le deuxième bord latéral étant opposé au premier bord latéral; le capteur de température (21; 121) couplé au dispositif de chauffage en un endroit situé entre les première et deuxième barres omnibus, dans laquelle le dispositif de chauffage se trouvera en contact conductif avec un corps lorsque la couverture est drapée sur le corps; le capteur de température fournissant une entrée au dispositif de commande de température (232), le dispositif de commande étant apte à commander une fourniture de puissance aux première et deuxième barres omnibus, la fourniture de puissance étant basée sur une température détectée par le capteur de température; et au moins un super-capteur de sur-température (41) couplé au dispositif de chauffage entre les première et deuxième barres omnibus, ledit moins un super-capteur de sur-température étant apte à interrompre la fourniture de puissance aux première et deuxième barres omnibus lorsqu'une température détectée par ledit au moins un super-capteur de sur-température dépasse une température prescrite.

8. Couverture (100 ; 200) selon la revendication 7, dans laquelle :

ledit au moins un super-capteur de sur-température (41) comprend une pluralité de super-capteurs de sur-température reliés en série les uns aux autres et présentant chacun une augmentation de résistance significative à des températures prescrites au-dessus d'une plage de fonctionnement normale du dispositif de chauffage.

9. Couverture (100 ; 200) selon la revendication 1, comprenant en outre :

une première barre omnibus conductrice (15) disposée le long d'un premier bord latéral (11) du dispositif de chauffage (10) ;
une deuxième barre omnibus conductrice (15) disposée le long d'un deuxième bord latéral (12) du dispositif de chauffage, le deuxième bord latéral étant opposé au premier bord latéral ;
une première rangée de piqûres (145) connectant la première barre omnibus au dispositif de chauffage ;
une deuxième rangée de piqûres (145) connectant la deuxième barre omnibus au dispositif de chauffage ;
les première et deuxième rangées de piqûres comprenant chacune un fil électriquement conducteur ;
les première et deuxième barres omnibus étant aptes à être couplées à la source de puissance (234) pour alimenter le dispositif de chauffage ;
un premier élément électriquement isolant (18) interposé entre la première barre omnibus et le dispositif de chauffage et fixé entre ceux-ci par la première rangée de piqûres ;
un deuxième élément électriquement isolant (18) interposé entre la deuxième barre omnibus et le dispositif de chauffage et fixé entre ceux-ci par la deuxième rangée de piqûres ; et
les premier et deuxième éléments isolants empêchant tout contact électrique direct entre les première et deuxième barres omnibus respectives et le dispositif de chauffage.

10. Couverture (100 ; 200) selon la revendication 1, comprenant en outre :

une première barre omnibus conductrice (15) disposée le long d'un premier bord (11) du dispositif de chauffage (10) et s'étendant au-delà des première (101) et deuxième (102) extrémités du dispositif de chauffage pour se terminer juste au-delà de chacune des extrémités, le premier bord s'étendant entre les première et deuxième extrémités du dispositif de chauffage ;
une deuxième barre omnibus conductrice (15) disposée le long d'un deuxième bord (12) du dispositif de chauffage et s'étendant au-delà des première et deuxième extrémités du dispositif de chauffage pour se terminer juste au-delà de chacune des extrémités, le deuxième bord étant opposé au premier bord et s'étendant entre les première et deuxième extrémités du dispositif de chauffage ;
au moins une première rangée de piqûres (145) couplant la première barre omnibus au dispositif de chauffage, ladite au moins une première rangée de piqûres s'étendant le long de la première barre omnibus ;
au moins une deuxième rangée de piqûres (145) couplant la deuxième barre omnibus au dispositif de chauffage, ladite au moins une deuxième rangée de piqûres s'étendant le long de la deuxième barre omnibus ; et
les première et deuxième barres omnibus étant aptes à être couplées à la source de puissance (234) pour alimenter l'élément chauffant du dispositif de chauffage de tissu conducteur.

11. Couverture (100 ; 200) selon la revendication 1, comprenant en outre :

une première barre omnibus conductrice (15) présentant un premier côté disposé contre le dispositif de chauffage (10), la barre omnibus s'étendant le long d'un premier bord (11) du dispositif de chauffage et présentant le premier bord du dispositif de chauffage replié sur celui-ci afin de le disposer contre un deuxième côté de la première barre omnibus ;
une deuxième barre omnibus conductrice (15) présentant un premier côté disposé contre le dispositif de chauffage, la barre omnibus s'étendant le long d'un deuxième bord (12) du dispositif de chauffage, opposé au premier bord, et présentant le deuxième bord du dispositif de chauffage replié sur celui-ci afin de le disposer contre un deuxième côté de la deuxième barre omnibus ;
au moins une première rangée de piqûres (145) couplant la première barre omnibus au dispositif de chauffage en s'étendant à travers le premier bord replié du dispositif de chauffage, la première barre omnibus, et le dispositif de chauffage disposé contre le premier côté de la première barre omnibus ;
au moins une deuxième rangée de piqûres (145) couplant la deuxième barre omnibus au dispositif de chauffage en s'étendant à travers le deuxième bord replié du dispositif de chauffage, la deuxième barre omnibus, et le dispositif de chauffage disposé contre le premier côté de la deuxième barre omnibus; et

les première et deuxième barres omnibus étant aptes à être couplées à la source de puissance (234) pour alimenter le dispositif de chauffage de tissu conducteur.

**12.** Couverture (100; 200) selon la revendication 1, comprenant en outre:

une première barre omnibus conductrice (15) disposée le long d'un premier bord (11) du dispositif de chauffage (10);
une deuxième barre omnibus conductrice (15) disposée le long d'un deuxième bord (12) du dispositif de chauffage, opposé au premier bord;
au moins une première rangée de piqûres (145) couplant la première barre omnibus au dispositif de chauffage, ladite au moins une première rangée de piqûres s'étendant le long de la première barre omnibus;
un premier ruban de matériau conducteur interposé entre la première barre omnibus et le dispositif de chauffage;
au moins une deuxième rangée de piqûres (145) couplant la deuxième barre omnibus au dispositif de chauffage, ladite au moins une deuxième rangée de piqûres s'étendant le long de la deuxième barre omnibus; et
un deuxième ruban de matériau conducteur interposé entre la deuxième barre omnibus et le dispositif de chauffage;
les première et deuxième barres omnibus étant aptes à être couplées à la source de puissance (234) pour alimenter le dispositif de chauffage de tissu conducteur.

**13.** Couverture (100; 200) selon la revendication 1, comprenant en outre:

une première barre omnibus conductrice (15) disposée le long d'un premier bord (11) du dispositif de chauffage (10);
une deuxième barre omnibus conductrice (15) disposée le long d'un deuxième bord (12) du dispositif de chauffage, opposé au premier bord;
au moins deux premières rangées de piqûres (145) couplant la première barre omnibus au dispositif de chauffage, lesdites au moins deux premières rangées de piqûres s'étendant le long de la première barre omnibus et comprenant un fil électriquement conducteur; et
au moins deux deuxièmes rangées de piqûres (145) couplant la deuxième barre omnibus au dispositif de chauffage, lesdites au moins deux deuxièmes rangées de piqûres s'étendant le long de la deuxième barre omnibus et comprenant un fil électriquement conducteur; et
les première et deuxième barres omnibus étant aptes à être couplées à la source de puissance (234) pour alimenter le dispositif de chauffage.

**14.** Couverture (100; 200) selon la revendication 1, dans laquelle:

le dispositif de chauffage (10) comprend un des éléments suivants: du carbone; ou un matériau non conducteur revêtu d'un matériau conducteur tel que le carbone, le polypyrrole, de l'encre métallique ou de l'encre carbonisée.

Figure 1C

Figure 1B

Figure 1A

Figure 1D

Figure 2B

Figure 2A

Figure 2C

EP 2 062 460 B1

Figure 2D

Figure 3A

Figure 3B

Figure 3D

Figure 3C

Figure 4A

Figure 4B

Figure 4C

EP 2 062 460 B1

Figure 4D

**EP 2 062 460 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4149066 A
- US 4186294 A
- US 3808403 A
- GB 969253 A
- US 20020117494 A
- WO 9925155 A

**Non-patent literature cited in the description**

- **STOLL ; GREENE.** Relationship between pain and tissue damage due to thermal radiation. *J. Applied Physiology,* 1959, vol. 14 (3), 373-382
- **MORITZ ; HENRIQUES.** Studies of thermal injury: The relative importance of time and surface temperature in the causation of cutaneous bums. *Am. J. Pathology,* 1947, vol. 23, 695-720